(19) Europäisches Patentamt
European Patent Office
Office européen des brevets

(11) **EP 4 138 083 A1**

(12) **EUROPEAN PATENT APPLICATION**
published in accordance with Art. 153(4) EPC

(43) Date of publication:
**22.02.2023 Bulletin 2023/08**

(21) Application number: **21788540.9**

(22) Date of filing: **06.04.2021**

(51) International Patent Classification (IPC):
**G16B 40/20** (2019.01)        **G16B 20/40** (2019.01)

(52) Cooperative Patent Classification (CPC):
**G16B 20/40; G16B 40/20**

(86) International application number:
**PCT/KR2021/004293**

(87) International publication number:
**WO 2021/210838 (21.10.2021 Gazette 2021/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.04.2020 KR 20200045382**

(71) Applicant: **Clinomics Inc.**
**Ulsan 44919 (KR)**

(72) Inventors:
• **BHAK, Jong Hwa**
**Ulju-gun Ulsan 44936 (KR)**
• **KIM, Byung Chul**
**Suwon-si Gyeonggi-do 16713 (KR)**
• **CHO, Yun Sung**
**Yongin-si Gyeonggi-do 16953 (KR)**
• **LEE, Hwang Yeol**
**Yongin-si Gyeonggi-do 16953 (KR)**

(74) Representative: **Frenkel, Matthias Alexander**
**Wuesthoff & Wuesthoff**
**Patentanwälte PartG mbB**
**Schweigerstraße 2**
**81541 München (DE)**

(54) **METHOD AND SYSTEM FOR PREDICTING BIOLOGICAL AGE ON BASIS OF VARIOUS OMICS DATA ANALYSES**

(57)     A system for predicting biological age on the basis of various omics data analyses, according to one embodiment of the present invention, comprises: a test sample collection unit for collecting a plurality of genetic test samples including DNA and/or RNA of a subject; a test sample analysis unit for analyzing a plurality of types of omics data from each of the plurality of genetic test samples; a preprocessing execution unit for preprocessing the omics data analyzed through the test sample analysis unit; an association analysis unit for performing an association analysis on the basis of the omics type of data for each omics area converted through the preprocessing execution unit; and an age prediction unit for predicting the age of the subject on the basis of the analyzed result of the association analysis unit and the data for each omics area.

Fig. 1

**Description**

**[Technical Field]**

The present invention relates to a method and system for predicting age based on analysis of various omics data, and more specifically, to a method for predicting aging or biological age using integrated information such as DNA methylation, mRNA expression level and telomere length, which acquires and comprehensively analyzes various omics data related to telomere length, DNA methylation, mRNA expression level, etc. from the specimen sample (e.g., blood) from a subject to predict the biological age of the subject and classify and analyze the degree of aging based on each omics data and a system for performing the same.

**[Background Arts]**

Biological age refers to the age quantified by comprehensively evaluating the overall health status and the degree of aging. In predicting such aging/biological age, the method using telomere length has been generally used. Biomarkers and combinations thereof are being developed to predict age based on DNA methylation or gene expression levels that change significantly with age.

[Prior art document]

US 20030148350 A1

**[Disclosure]**

**[Technical Problem]**

The issue to be addressed by the present invention is to provide a method and system for predicting biological age based on various omics data analysis that can solve the problems of the prior art.

**[Technical Solution]**

A system for predicting biological age based on various omics data analysis according to an embodiment of the present invention for addressing the above issues comprises: a test sample collection unit for collecting a plurality of genetic test samples, including at least one of DNA and RNA of a subject; a test sample analysis unit for analyzing a plurality of types of omics data from each of the plurality of genetic test samples; a preprocessing unit for preprocessing the omics data analyzed through the test sample analysis unit; an association analysis unit for performing an association analysis based on the omics type of data for each omics area converted through the preprocessing unit; and an age prediction unit for predicting the subject's age based on the analyzed result of the association analysis unit and the data for each omics area.

A method for predicting biological age based on various omics data analysis according to an embodiment of the present invention for addressing the above issues comprises steps of collecting a plurality of genetic test samples, including at least one of DNA and RNA of a subject in a test sample collection unit; analyzing a plurality of types of omics data from each of the plurality of genetic test samples in a test sample analysis unit; preprocessing the omics data analyzed through the test sample analysis unit in a preprocessing unit; performing an association analysis based on each omics type of data for each omics area converted through the preprocessing unit in an association analysis unit; predicting the age of a subject based on the analysis result of the association analysis unit and the data for each omics area in the age prediction unit.

**[Advantageous Effects]**

The method and system for predicting biological age based on various omics data analysis according to an embodiment of the present invention a reused to combine and reflect markers of various omics regions in the biological age prediction model, thereby having the advantage of being able to offset the existing error in individual omics area. It allows more accurate age prediction and distinguishing and interpreting the influence (or the degree of aging) of each omics area on the integratedly predicted biological age (the current degree of aging of the subject).

That is, through the combination of three omics data, such as the genome (telomere length), exogenous (methylation), and transcript (gene expression) of samples such as human blood: 1) the age prediction accuracy can be increased by offsetting the noise;2) The biological age (degree of aging) of the subject can be analyzed by dividing it by omics area.

**[Description of Drawings]**

Fig. 1 is a block diagram of a system for predicting biological age based on analysis of various omics data according to an embodiment of the present invention.

Figs. 2a to 2d are flowcharts illustrating a method for predicting biological age based on analysis of various omics data.

Fig. 3 is a graph showing the correlation between the biological age and the actual age based on linear regression using the telomere length.

Fig. 4 is a graph showing the correlation between biological age and actual age based on linear regression using sixteen methylation markers.

Fig. 5 is a graph showing the correlation between the biological age and the actual age based on linear regression using eighteen gene expression markers.

Fig. 6 is a graph showing the correlation between the omics-integrated biological age and actual age based on linear regression combining telomere length, methylation marker, and gene expression marker presented in the present invention.

Fig. 7 is a graph showing the correlation between omics-integrated biological age and actual age by summing the telomere-based age, methylation-based age, and gene expression-based age presented in the present invention by weights (weighting coefficient of determination) for each omics area.

Fig. 8 is a graph showing the correlation between the biological age and the actual age based on linear regression using four methylation markers.

Fig. 9 is a graph showing the correlation between living age and actual age based on linear regression using four gene expression markers.

Fig. 10 is a graph showing the correlation between the omics-integrated biological age and actual age based on linear regression combining telomere length, methylation, and gene expression markers presented in the present invention.

Fig. 11 is a graph showing the correlation between omics-integrated biological age and actual age by summing the telomere-based age, methylation-based age, and gene expression-based age presented in the present invention by weights (weighting coefficient of determination) for each omics area.

Fig. 12 is a graph showing the correlation between biological age and actual age based on an artificial neural network using telomere length.

Fig. 13 is a graph showing the correlation between biological age and actual age based on an artificial neural network using sixteen methylation markers.

Fig. 14 is a graph showing the correlation between biological age and actual age based on an artificial neural network using eighteen gene expression markers.

Fig. 15 is a graph showing the correlation between the biological age and the actual age of the artificial neural network-based omics integration combining the telomere length, methylation marker, and gene expression marker presented in the present invention.

Fig. 16 is a graph showing the correlation between an omics-integrated biological age and the actual age obtained by summing the telomere-based age, methylation-based age, and gene expression-based age using the artificial neural network presented in the present invention by weights (weighting coefficient of determination) for each omics area.

Fig. 17 is a graph showing the correlation between omics-integrated biological age and actual age by summing the telomere-based age, methylation-based age, and gene expression-based age presented in the present invention by weights (weighting significance) for each omics area.

Fig. 18 is a graph showing the correlation between omics-integrated biological age and actual age by summing the telomere-based age, methylation-based age, and gene expression-based age presented in the present invention by weights (weighting mean error) for each omics area.

**[Mode for Invention]**

Hereinafter, embodiments of the present invention are described in detail with reference to the accompanying drawings so that those of ordinary skill in the art can easily carry out the present invention. However, the present invention may be embodied in several different forms and is not limited to the embodiments described herein. Further, in order to clearly explain the present invention in the drawings, parts irrelevant to the description are excluded, and similar reference numerals are assigned to similar parts throughout the specification.

Throughout the specification, when a part is "connected" with another part, it is not only "directly connected" but also "electrically connected" with another element interposed therebetween. Further, when a part "includes" a certain component, it means that other components may be further included, rather than excluding other components, unless otherwise stated, and it is to be understood that the existence or addition of one or more other features, numbers, steps, operations, components, parts, or combinations thereof is not precluded in advance.

The terms "about," "substantially," etc. related to the extent used throughout the specification are used in a sense at or close to the numerical value when the manufacturing and material tolerances inherent in the stated meaning are presented and are used to prevent an unscrupulous infringer from using the disclosure in which exact or absolute values are mentioned to aid the understanding of the present invention. As used throughout the specification of the present invention, the term "step of (to)" or "step of" does not mean "step for."

In this specification, a "unit" includes a unit implemented by hardware, a unit implemented by software, and a unit realized using both. In addition, one unit may be implemented using two or more hardware, and two or more units may be implemented with one hardware.

In this specification, some of the operations or functions described as being performed by the terminal, apparatus, or device may be performed instead of in a server connected to the terminal, apparatus, or device. Similarly, some of the operations or functions described as being performed by the server may also be performed in a terminal, apparatus, or device connected to the server.

In this specification, some of the operations or functions described as mapping or matching with the terminal may be interpreted to mean mapping or matching the terminal's unique number or personal identification information, which is identifying data of the terminal.

Hereinafter, the present invention is described in detail with reference to the accompanying drawings.

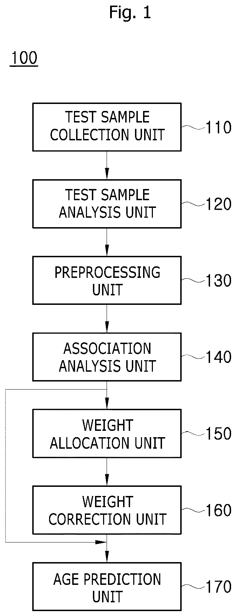

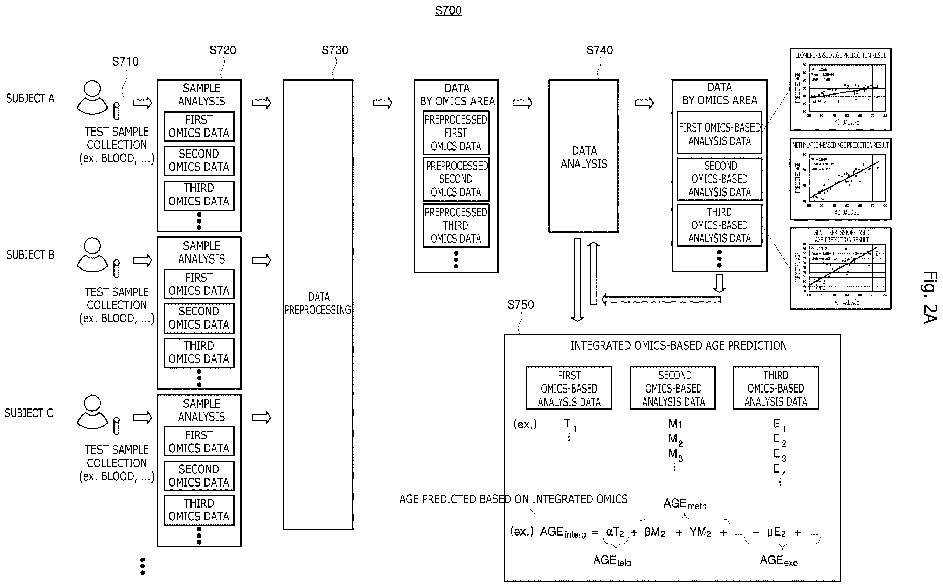

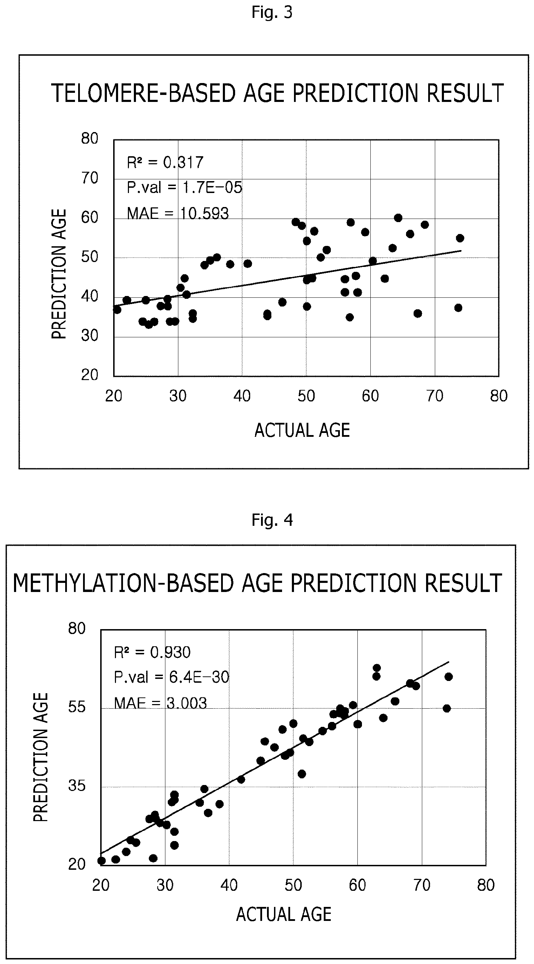

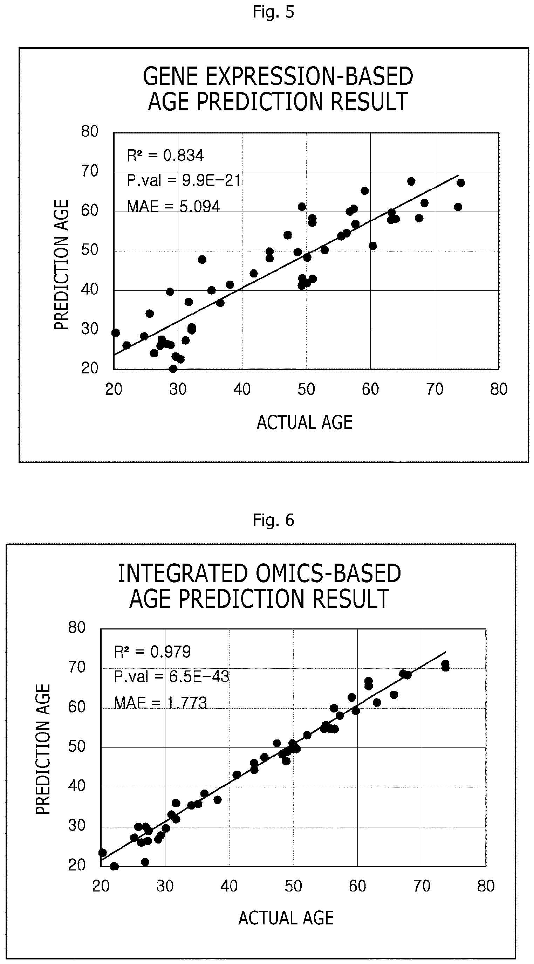

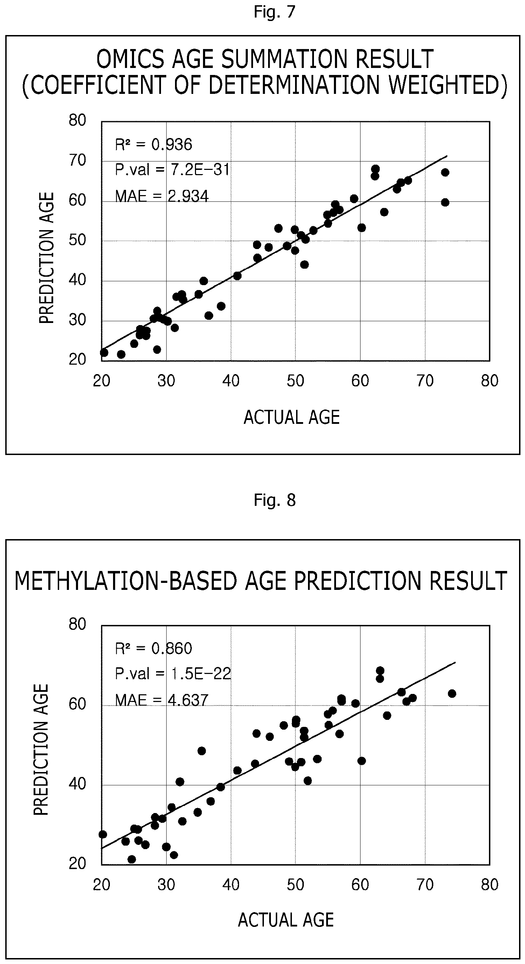

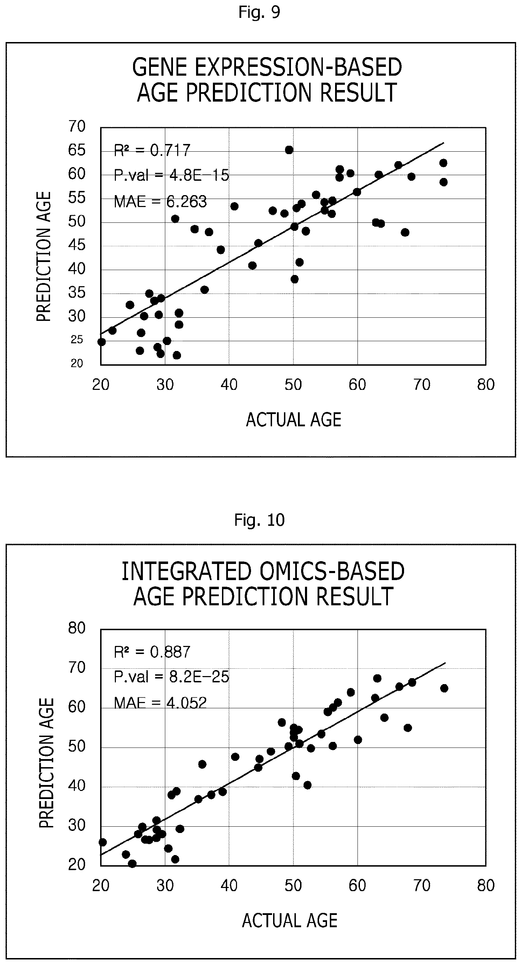

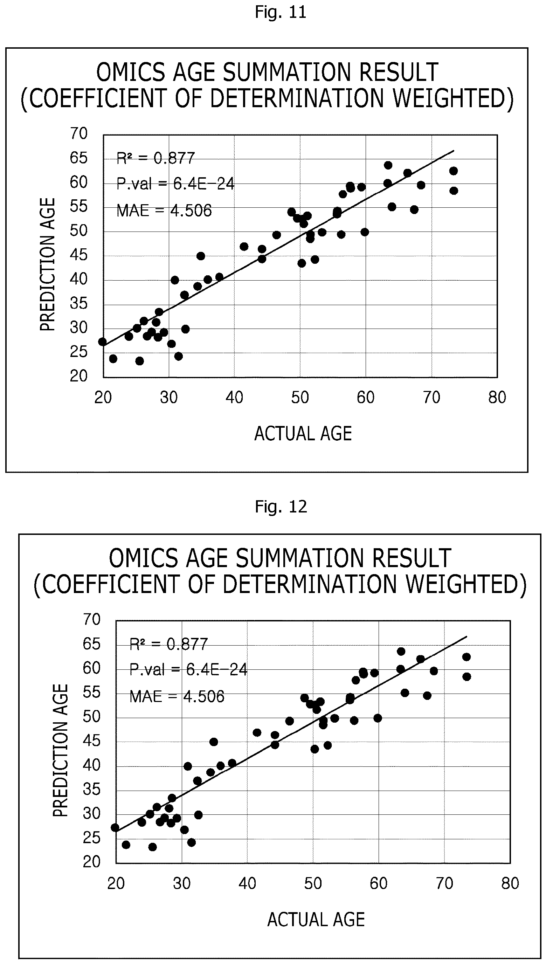

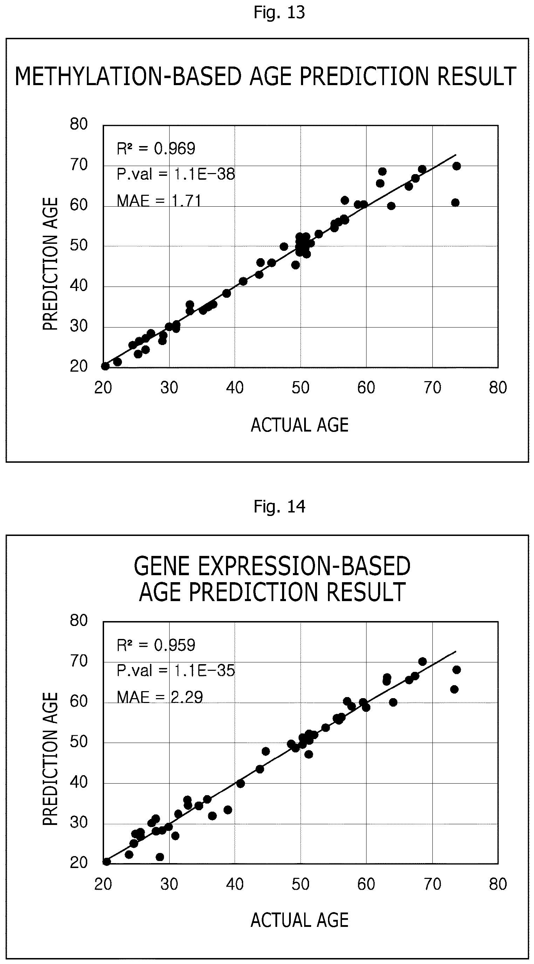

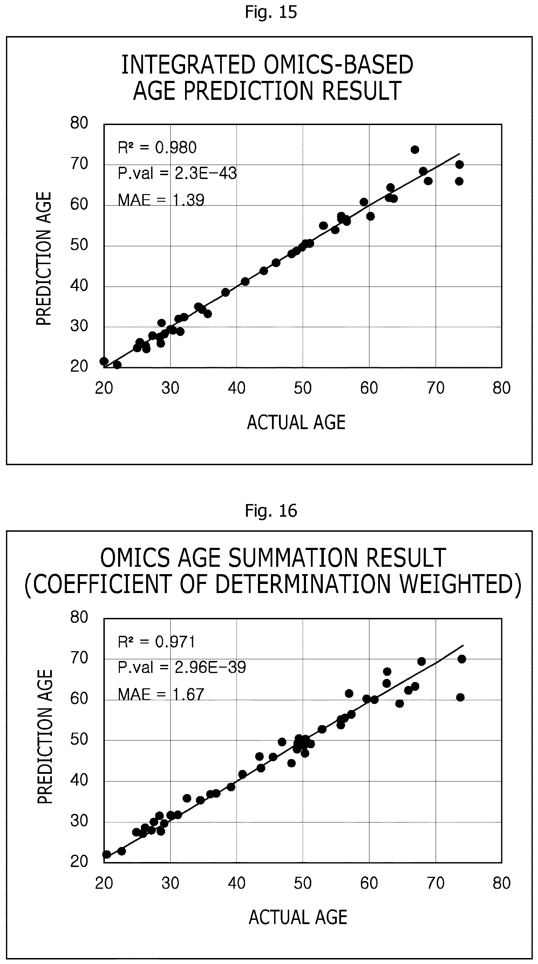

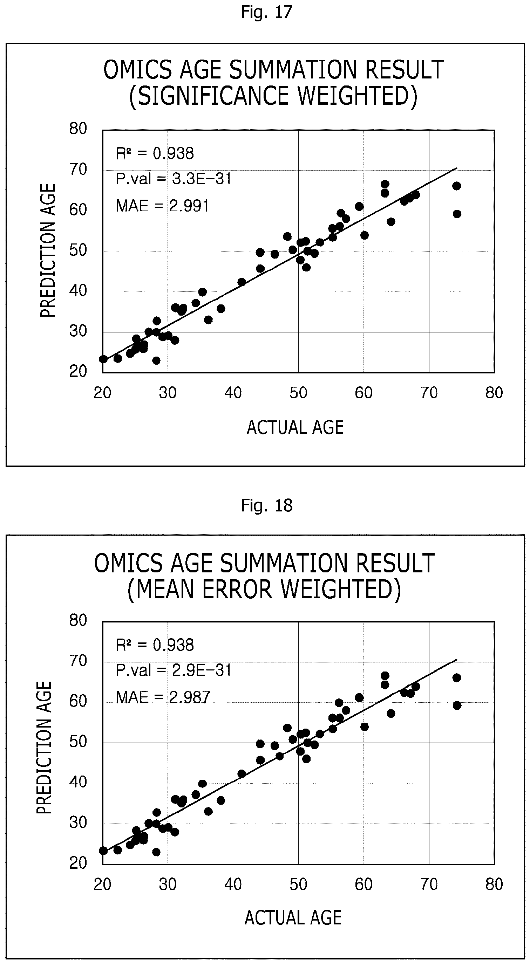

Fig. 1 is a block diagram of a system for predicting biological age based on analysis of various omics data according to an embodiment of the present invention. Figs. 2a to 2d are flowcharts illustrating a method for predicting biological age based on analysis of various omics data. Fig. 3 is a graph showing the correlation between the biological age and the actual age based on linear regression using the telomere length. Fig. 4 is a graph showing the correlation between biological age and actual age based on linear regression using sixteen methylation markers. Fig. 5 is a graph showing the correlation between the biological age and the actual age based on linear regression using eighteen gene expression markers. Fig. 6 is a graph showing the correlation between the omics-integrated biological age and actual age based on linear regression combining telomere length, methylation marker, and gene expression marker presented in the present invention. Fig. 7 is a graph showing the correlation between omics-integrated biological age and actual age by summing the telomere-based age, methylation-based age, and gene expression-based age presented in the present invention by weights (weighting coefficient of determination) for each omics area. Fig. 8 is a graph showing the correlation between the biological age and the actual age based on linear regression using four methylation markers. Fig. 9 is a graph showing the correlation between living age and actual age based on linear regression using four gene expression markers. Fig. 10 is a graph showing the correlation between the omics-integrated biological age and actual age based on linear regression combining telomere length, methylation, and gene expression markers presented in the present invention. Fig. 11 is a graph showing the correlation between omics-integrated biological age and actual age by summing the telomere-based age, methylation-based age, and gene expression-based age presented in the present invention by weights (weighting coefficient of determination) for each omics area. Fig. 12 is a graph showing the correlation between biological age and actual age based on an artificial neural network using telomere length. Fig. 13 is a graph showing the correlation between biological age and actual age based on an artificial neural network using sixteen methylation markers. Fig. 14 is a graph showing the correlation between biological age and actual age based on an artificial neural network using eighteen gene expression markers. Fig. 15 is a graph showing the correlation between the biological age and the actual age of the artificial neural network-based omics integration combining the telomere length, methylation marker, and gene expression marker presented in the present invention. Fig. 16 is a graph showing the correlation between an omics-integrated biological age and the actual age obtained by summing the telomere-based age, methylation-based age, and gene expression-based age using the artificial neural network presented in the present invention by weights (weighting coefficient of determination) for each omics area. Fig. 17 is a graph showing the correlation between omics-integrated biological age and actual age by summing the telomere-based age, methylation-based age, and gene expression-based age presented in the present invention by weights (weighting significance) for each omics area. Fig. 18 is a graph showing the correlation between omics-integrated biological age and actual age by summing the telomere-based age, methylation-based age, and gene expression-based age presented in the present invention by weights (weighting mean error) for each omics area.

First, as shown in Fig. 1, the system for predicting biological age 100 based on the analysis of various omics data according to an embodiment of the present invention acquires various omics information (e.g., telomere length, DNA methylation level, gene expression level, etc.) from test samples (e.g., blood) and integratedly analyze each acquired omics information (e.g., multiple linear regression analysis, weighting for each omics region) to measure (predict) biological age more accurately than before.

More specifically, the system for predicting biological age 100 based on the analysis of various omics data of the present invention is a test sample collection unit 110, a test sample analysis unit 120, a preprocessing unit 130, an association analysis unit 140, a weight allocation unit 150, a weight correction unit 160, and an age prediction unit 170.

The test sample collection unit 110 includes a configuration for collecting a plurality of genetic test samples containing the DNA and RNA of the subject, that is, a configuration for collecting and then classifying a plurality of aging biomarker test samples.

Here, the aging biomarker may be information on measuring telomere length by collecting DNA from blood samples of various age groups, performing DMR analysis through methyl-seq or chip experiment, and performing DEG analysis through RNA-seq or microarray experiments on collected RNA and the like.

Further, each aging biomarker test sample is classified into learning data and test data, and the classified learning data and test data are used for age prediction of each omics area marker, integrated omics analysis, and predicted age weight summation analysis.

Next, the test sample analysis unit 120 is configured to analyze a plurality of types of omics data from each of the plurality of genetic test samples. That is, it may be a configuration for analyzing an omics area, including telomere length information, DNA methylation information, and gene expression level for each gene from the plurality of genetic test samples.

Specifically, the test sample analysis unit 120 comprises a telomere length measurement unit, a methylation marker analysis and filtering unit, and a gene expression marker analysis and filtering unit.

The telomere length measurement unit is configured to measure the relative length of telomeres compared to a single copy gene using the qPCR, TRF, or Q-FISH method, in which the fluorescence detection limit cycle number (Ct) for each concentration is measured from a standard oligomer sample of known length. Then the total telomere length is obtained by dividing the Ct value of the telomeres by the Ct value of the reference gene, and the absolute length of the telomeres is measured by dividing this by the number of telomeres in the human genome.

For reference, the above-described method for measuring telomere length is only an example, and various conventional methods for measuring telomere length may be applied.

Next, the methylation marker analysis and filtering unit map the methylation raw data obtained using DMR analysis, etc. through experiments such as Methyl-seq, chip, etc. to a human genome map (human reference), thereby obtaining the methylation degree (hereinafter, "beta value") by location of each test sample and selects areas in which beta values increase or decrease according to age in each test sample using DMR analysis.

Next, the gene expression marker analysis and filtering unit map the gene expression raw data obtained through experiments such as RNA-seq and microarray to the human genome map (human reference) to calculate the expression level for each gene in each test sample, remove the batch effect according to gender/lifestyle, etc. from the calculated gene expression level, and then select genes whose expression level increases or decreases according to age in each test sample using DEG analysis and the like.

Next, the preprocessing unit 130 is configured to perform preprocessing on the omics data analyzed through the test sample analysis unit 120.

More specifically, the preprocessing unit 130 converts beta values and expression level values of selected methylation markers and gene expression markers, and telomere length into percentiles in the range of 0 to 1 for the application of multiple linear regression analysis or artificial neural network-based regression analysis.

Next, the association analysis unit 140 performs an association analysis based on each omics type of data for each omics area converted through the preprocessing unit 130. More specifically, the association analysis unit 140 uses multiple linear regression analysis or artificial neural network-based regression analysis to calculate each coefficient value of the independent variable in a regression model with the preprocessed value of the biomarker for each omics area converted as an independent variable and biological age as a dependent variable. Through the calculated coefficients, the association between the biological age and the actual age for each area predicted from the preprocessed value of each omics area biomarker is analyzed, and the analyzed association may be one of the coefficients of determination ( $R_x^2$ ), significance *(PVAL$_x$)*, and mean absolute error *(MAE$_x$)*.

Next, the weight allocation unit 150 may be configured to assign a weight to each type of omics data based on any one of the associations (coefficient of determination, significance, and mean absolute error) analyzed through the association analysis unit.

The weight allocation unit 150 calculates the weight *(W$_x$)* for the coefficient of determination ( $R_x^2$ ), significance *(PVAL$_x$)*, and mean absolute error *(MAE$_x$)* of each omics area using the following equations.

$$W_x = 1/(1 - R_x^2) \ldots\ldots \text{(Weight equation for coefficient of determination)}$$

$$W_x = \log(\text{PVAL}_x)*(-1)\dots\dots(\text{Weight equation for significance})$$

$$W_x = 1/\text{mae}_{x,\text{rev}}\dots\dots(\text{Weight equation for mean absolute error})$$

The weight correction unit 160 may be configured to obtain a correction weight ($W_{x,rev}$) by exponentiating a weighted average value ($W_{avg}$) for each area of the weights given to each type of omics data using the following equations.

$$W_{x,rev} = W_{avg}{}^{(W_x/W_{avg})}\dots\dots(\text{Weight correction equation})$$

Meanwhile, the weight correction unit 160 may obtain distribution correction ($mae_{x,rev}$) by the average age ($AGE_{avg}$) of the sample group to relatively reflect the mean absolute error compared to the actual age distribution before weight correction for the mean absolute error ($MAE_x$) of each omics area through the following equation.

$$mae_{x,rev} = MAE_x/AGE_{avg}$$

Next, the age prediction unit 170 is configured to predict the subject's age based on the analysis result of the association analysis unit and the data for each omics area and may predict the subject's age through the following equation.

$$AGE_{integ} = \frac{\Sigma)AGE_X * W_{X,rev})}{\Sigma W_{X,rev}}$$

(Biological age AGE$_{integ}$ prediction equation)

That is, the age prediction unit 170 is configured to calculate the weights of each omics area using any one of the coefficients of determination, significance, and mean absolute error for the age of individual omics data and then predict biological age or aging state by comparing the sum of the age inferred from the individual omics according to the weight.

Hereinafter, with reference to the drawings, the first comparative example compares the predictive power of the linear regression-based biological age to the actual age using telomere length, sixteen methylation markers, and eighteen gene expression markers through the configurations disclosed herein are briefly described.

1) Omics integrated multiple linear regression analysis

In the first comparative example, the association analysis unit 140 of the present application performs multiple linear regression analysis and omics integration analysis for each area using sixteen methylation markers based on preselected adjusted p-value <1.0e-30 and eighteen gene expression markers based on adj.pval<5.0e-02 along with the telomere length.

2) Summation analysis of biological age weights by omics area (weighted coefficient of determination)

The association analysis unit 140 of the present application obtains the coefficient of determination ( $R_x^2$ ) for the actual age of the sample of the biological age predicted for each area from multiple linear regression analysis using the markers of each omics area.

The weight allocation unit 150 of the present application calculates the weight ($W_x$) of each omics area as in Equation 1 in order to give greater weight to the omics region having a large coefficient of determination.

[Equation 1]

$$W_x = 1/(1-R_x^2)$$

Further, when the difference in the coefficient of determination for the actual age of the biological age between each omics area is large, the weight correction unit 160 of the present application calculates a corrected weight value ($W_{x,rev}$) through exponentiation of the average weight value ($W_{avg}$) of each omics area as shown in Equation 2 in order to emphasize and reflect the age of the omics area with high reliability in the weight ($W_x$) for each area.

The age prediction unit 170 of the present application calculates an omics-integrated biological age ($AGE_{integ}$) by applying and summing a weight for each omics area to the predicted age ($AGE_x$) for each area, as shown in Equation 3, and summing them.

[Equation 2]

$$W_{x,rev} = W_{avg}{}^{(Wx/Wavg)}$$

[Equation 3]

$$AGE_{integ} = \frac{\sum AGE_X * W_{X,rev}}{\sum W_{X,rev}}$$

Table 1 below shows the coefficient of determination, weight, and correction weight of each omics area and Table 2 compares the predicted value of omics-integrated biological age by summing the age for each omics area and weights for each omics area and the actual age.

[Table 1]

|  | Telomeres | Methylation | Gene expression |
|---|---|---|---|
| Coefficient of determination ( $R_x^2$ ) | 0.317 | 0.930 | 0.834 |
| Weight $W_x$ | 1.46 | 14.25 | 6.02 |
| Corrected weight $W_{x,rev}$ | 1.49 | 49.19 | 5.19 |

Table 2

| $AGE_{telo}$ | $W_{telo}$ | $AGE_{meth}$ | $W_{meth}$ | $AGE_{exp}$ | $W_{exp}$ | $AGE_{interg}$ | Actual age |
|---|---|---|---|---|---|---|---|
| 38.63 | 1.49 | 21.31 | 49.19 | 26.46 | 5.19 | 22.25 | 22 |
| 34.38 |  | 46.24 |  | 47.15 |  | 46.00 | 44 |
| 57.15 |  | 66.77 |  | 67.77 |  | 66.61 | 74 |

Table 3 compares the omics integrated regression analysis of biological age and age-weighted summation of omics integrated biological age prediction results for each omics area compared to individual omics biological age prediction.

[Table 3]

|  | Telomere-based age prediction | Methylation-based age prediction | Gene expression-based age prediction | integrated omics-based on age prediction | Result of age-weighted summation by omics |
|---|---|---|---|---|---|
| Coefficient of determination ($R^2$) | 0.317 | 0.930 | 0.834 | 0.979 | 0.936 |
| Significance (P-val) | 1.7E-05 | 6.4E-30 | 9.9E-21 | 6.5E-43 | 7.2E-31 |
| Mean absolute error (MAE) | 10.593 | 3.003 | 5.094 | 1.773 | 2.934 |

Referring to Table 3, it can be shown that the omics integrated biological age prediction by omics integrated regression analysis or age weight summation for each omics area is closer to the actual age of the sample in terms of coefficient of determination and significance, and the mean error (MAE) is smaller compared to the age-predicted through multiple linear regression analysis from individual omics.

Hereinafter, with reference to the drawings, the second comparative example comparing the predictive power of the linear regression-based biological age to the actual age using the telomere length, four methylation markers, and four gene expression markers through the configurations disclosed herein is briefly described.

1) Omics integrated multiple linear regression analysis

In the second comparative example, the association analysis unit 140 of the present application performs multiple linear regression analysis and omics integration analysis for each area by selecting four methylation markers based on adj.Pval<1.0e-30 and the absolute value of the association between the marker and the actual age | R | > 0.75 and four gene expression markers based on adj.Pval<1.0e-04 along with the telomere length.

2) Summation analysis of biological age weights by omics area (weighted coefficient of determination)

It is applied in the same manner as in the first comparative example. Table 4 below shows the coefficient of determination, weight, and correction weight of each omics area and Table 5 compares the predicted value of omics-integrated biological age by summing the age for each omics area and weights for each omics area and the actual age.

Table 4

| | Telomeres | Methylation | Gene expression |
|---|---|---|---|
| Coefficient of a determination ( $R_x^2$ ) | 0.310 | 0.860 | 0.717 |
| Weight $W_x$ | 1.46 | 7.14 | 3.54 |
| Corrected weight $W_{x,rev}$ | 1.66 | 11.78 | 3.39 |

Table 5

| $AGE_{telo}$ | $W_{telo}$ | $AGE_{meth}$ | $W_{meth}$ | $AGE_{exp}$ | $W_{exp}$ | $AGE_{interg}$ | Actual age |
|---|---|---|---|---|---|---|---|
| 38.63 | 1.66 | 19.04 | 11.78 | 27.73 | 3.39 | 23.94 | 22 |
| 34.38 | | 45.20 | | 45.58 | | 44.01 | 44 |
| 36.77 | | 63.13 | | 57.61 | | 58.34 | 74 |

Table 6 compares the omics integrated regression analysis of biological age and age-weighted summation of omics integrated biological age prediction results for each omics area compared to individual omics biological age prediction.

[Table 6]

| | Telomere-based age prediction | Methylation-based age prediction | Gene expression-based age prediction | integrated omics-based on age prediction | Result of age-weighted summation by omics |
|---|---|---|---|---|---|
| Coefficient of determination ($R^2$) | 0.317 | 0.860 | 0.717 | 0.887 | 0.877 |
| Significance (P-val) | 1.7E-05 | 1.5E-22 | 4.8E-15 | 8.2E-25 | 6.4E-24 |
| Mean absolute error (MAE) | 10.593 | 4.637 | 6.263 | 4.052 | 4.506 |

Referring to Table 6, it can be shown that the omics integrated biological age prediction by omics integrated regression analysis or age weight summation for each omics area is closer to the actual age of the sample in terms of coefficient of determination and significance, and the mean error (MAE) is smaller compared to the age-predicted through multiple linear regression analysis from individual omics.

Hereinafter, with reference to the drawings, the third comparative example comparing the predictive power of the artificial neural network-based biological age to the actual age using the telomere length, sixteen methylation markers, and eighteen gene expression markers through the configurations disclosed herein is briefly described.

1) Omics integrated artificial neural network-based regression analysis

In the third comparative example, the association analysis unit 140 of the present application performs artificial neural network-based regression analysis and omics integration analysis for each area by selecting sixteen methylation markers based on adj.Pval<1.0e-30 and eighteen gene expression markers based on adj.Pval<5.0e-02 along with the telomere length.

2) Summation analysis of biological age weights by omics area (weighted coefficient of determination)

It is applied in the same manner as in the first comparative example.
Table 7 below shows the coefficient of determination, weight, and correction weight of each omics area and Table 8 compares the predicted value of omics-integrated biological age by summing the age for each omics area and weights for each omics area and the actual age.

Table 7

| | Telomeres | Methylation | Gene expression |
|---|---|---|---|
| Coefficient of determination ( $R_x^2$ ) | 0.309 | 0.969 | 0.959 |
| Weight $W_x$ | 1.45 | 32.40 | 24.45 |
| Corrected weight $W_{x,rev}$ | 1.25 | 140.86 | 41.82 |

Table 8

| $AGE_{telo}$ | $W_{telo}$ | $AGE_{meth}$ | $W_{meth}$ | $AGE_{exp}$ | $W_{exp}$ | $AGE_{interg}$ | Actual age |
|---|---|---|---|---|---|---|---|
| 41.29 | 1.25 | 21.18 | 140.86 | 19.62 | 41.82 | 20.96 | 22 |
| 34.46 | | 46.31 | | 47.53 | | 46.51 | 44 |
| 53.73 | | 70.24 | | 67.73 | | 69.56 | 74 |

Table 9 compares the omics integrated regression analysis, and age-weighted summation omics integrated biological age prediction results compared to artificial neural network-based individual omics biological age prediction.

[Table 9]

| | Telomere-based age prediction | Methylation-based age prediction | Gene expression-based age prediction | integrated omics-based on age prediction | Result of age-weighted summation by omics |
|---|---|---|---|---|---|
| Coefficient of determination ( $R^2$ ) | 0.309 | 0.969 | 0.959 | 0.979 | 0.971 |
| Significance (P-val) | 2.3E-05 | 1.1E-38 | 1.1E-35 | 6.5E-43 | 3.0E-39 |
| Mean absolute error (MAE) | 10.656 | 1.712 | 2.285 | 1.773 | 1.671 |

Referring to Table 9, it can be shown that the omics integrated biological age prediction by omics integrated regression analysis or age weight summation for each omics area is closer to the actual age of the sample in terms of coefficient of determination and significance, and the mean error (MAE) is smaller compared to the age predicted through artificial neural network-based regression analysis from individual omics.

Hereinafter, with reference to the drawings, the fourth comparative example compares the linear regression-based age prediction (weight scoring)using the telomere length, sixteen methylation markers, and eighteen gene expression markers through the configurations disclosed herein are described.

1) Omics integrated multiple linear regression analysis

In the fourth comparative example, the association analysis unit 140 of the present application performs multiple linear regression analysis and omics integration analysis for each area by selecting sixteen methylation markers based on adjusted p-value <1.0e-30 and eighteen gene expression markers based on adj.pval<5.0e-02 along with the telomere length.

2-1) Summation analysis of biological age weights by omics area (weighted significance)

The association analysis unit 140 of the present application obtains the significance ($PVAL_x$) between the biological age predicted for each area (x) from multiple linear regression analysis using the markers of each omics area and the sample's actual age.

The weight allocation unit 150 of the present application calculates the weight ($W_x$) as in Equation 4 to transform the significance scale distributed in a very small error range.

[Equation 4]

$$W_x = \log(PVAL_x)*(-1)$$

Further, when the difference in the significance between the biological age and the actual age between each omics area is large, the weight correction unit 160 of the present application calculates a corrected weight value ($W_{x,rev}$) as shown in Equation 5 through exponentiation of the average weight value ($W_{avg}$) of each omics area in order to emphasize and reflect the age of the omics area with high reliability in the weight ($W_x$) for each area.

The age prediction unit 170 of the present application calculates the biological age ($AGE_{integ}$) by summing the weights for each omics region.

[Equation 5]

$$W_{x,rev} = W_{avg}^{(W_x/W_{avg})}$$

[Equation 6]

$$AGE_{integ} = \frac{\Sigma)AGE_x * W_{x,rev})}{\Sigma W_{x,rev}}$$

Table 10 below shows the significance, weight, and correction weight of each omics area, and Table 11 compares the predicted value of omics-integrated biological age by summing the age for each omics area and weights for each omics area and the actual age.

[Table 10]

|  | Telomeres | Methylation | Gene expression |
|---|---|---|---|
| Significance ($PVAL_x$) | 1.7E-05 | 6.4E-30 | 9.9E-21 |
| Weight $W_x$ | 4.77 | 29.20 | 20.01 |
| Corrected weight $W_{x,rev}$ | 2.15 | 108.67 | 24.84 |

Table 11]

| AGE$_{telo}$ | W$_{telo}$ | AGE$_{meth}$ | W$_{meth}$ | AGE$_{exp}$ | W$_{exp}$ | AGE$_{interg}$ | Actual age |
|---|---|---|---|---|---|---|---|
| 38.63 | 2.15 | 21.31 | 108.67 | 26.46 | 24.84 | 22.53 | 22 |
| 34.38 | | 46.24 | | 47.15 | | 46.00 | 44 |
| 57.15 | | 66.77 | | 67.77 | | 66.81 | 74 |

2-2) Summation analysis of biological age weights by omics area (weighted mean error)

The association analysis unit 140 of the present application obtains mean absolute error (*MAEx*) between the biological age predicted for each area (x) from multiple linear regression analysis using the markers of each omics area and the sample's actual age.

The weight allocation unit 150 of the present application calculates the weight ($W_x$) of each omics area as in Equation 7 in order to give greater weight to the omics area with a small mean absolute error.

[Equation 7]

$$W_x = 1/mae_{x,rev}$$

Further, in order to relatively reflect the mean absolute error compared to the actual age distribution, distribution correction (*mae$_{x,rev}$*) by the average age (*AGE$_{avg}$*) of the sample group is applied as shown in Equation 8 below, when the difference in the mean absolute error between the biological age and the actual age between each omics area is large, the weight correction unit 160 of the present application calculates a corrected weight value ($W_{x,rev}$) as shown in Equation 9 through exponentiation of the average weight value ($W_{avg}$) of each omics area in order to emphasize and reflect the age of the omics area with high reliability in the weight *($W_x$)* for each area. Then, the integrated biological age (AGE$_{integ}$) is calculated by summing the correction weights for each omics area using Equation 10.

[Equation 8]

$$mae_{x,rev} = MAE_x/AGE_{avg}$$

[Equation 9]

$$W_{x,rev} = W_{avg}{}^{(W_x/Wavg)}$$

[Equation 10]

$$AGE_{integ} = \frac{\Sigma)AGE_x * W_{x,rev})}{\Sigma W_{x,rev}}$$

Table 12 below shows the mean absolute error. Correction means absolute error, weight, and correction weight of each omics area, and Table 13 compare the predicted value of omics-integrated biological age by summing the age for each omics area and weights for each omics area and the actual age.

Table 12]

| | Telomeres | Methylation | Gene expression |
|---|---|---|---|
| Mean absolute error (MAE$_x$) | 10.593 | 3.003 | 5.094 |
| Correction mean absolute error (mae$_{x,rev}$) | 0.237 | 0.067 | 0.114 |
| Weight W, | 4.22 | 14.89 | 8.78 |
| Corrected weight W$_{x,rev}$ | 2.75 | 35.57 | 8.21 |

[Table 13]

| AGE$_{telo}$ | W$_{telo}$ | AGE$_{meth}$ | W$_{meth}$ | AGE$_{exp}$ | W$_{exp}$ | AGE$_{interg}$ | Actual age |
|---|---|---|---|---|---|---|---|
| 38.63 | 2.75 | 21.31 | 35.57 | 26.46 | 8.21 | 23.24 | 22 |
| 34.38 | | 46.24 | | 47.15 | | 45.70 | 44 |
| 57.15 | | 66.77 | | 67.77 | | 66.38 | 74 |

Table 14 below compares the age-weighted summation of omics integrated biological age prediction results to which each weighting method is applied compared to individual omics biological age prediction.

[Table 14]

| | Telomere-based age prediction | Methylation-based age prediction | Gene expression-based age prediction | integrated omics-based on age prediction | Result of age-weighted summation by omics |
|---|---|---|---|---|---|
| Coefficient of determination ($R^2$) | 0.317 | 0.930 | 0.834 | 0.938 | 0.938 |
| Significance (P-val) | 1.7E-05 | 6.4E-30 | 9.9E-21 | 3.3E-31 | 2.9E-31 |
| Mean absolute error (MAE) | 10.593 | 3.003 | 5.094 | 2.991 | 2.987 |

Referring to Table 14, it can be seen that the omics-integrated biological age, which is weighted by scoring significance or mean error compared to the predicted age through regression analysis from individual omics, is closer to the actual age of the sample in terms of coefficient of determination and significance, and the mean error is smaller.

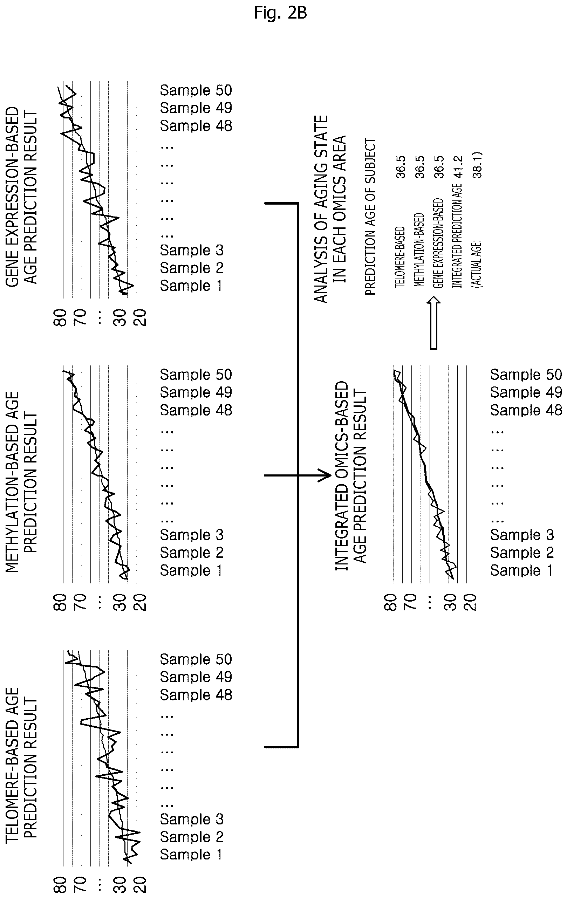

Hereinafter, a method for predicting biological age based on various omics data analysis according to the first embodiment of the present invention is described with reference to Figs. 2a and 2b.

The method S700 for predicting biological age based on various omics data analysis according to an embodiment of the present invention collects a plurality of genetic test samples, including DNA and RNA of a subject in the test sample collection unit 110 (S710), then analyzes a plurality of types of omics data (including at least one of telomere length, methylation, and gene expression) from each of the plurality of genetic test samples in the test sample analysis unit 120 (S720), and then preprocesses conversion of each marker value of the omics data analyzed through the test sample analysis unit 120 into a percentile value in the range of 0 to 1 in a preprocessing unit 130 (S730).

Thereafter, the method performs an association analysis based on the type of omics data for each omics area converted through the preprocessing unit 130 in the association analysis unit 140 (S740).

Process S740 is a process of analyzing the correlation between data for a plurality of omics areas using multiple linear regression analysis or artificial neural network-based regression analysis in which the analyzed correlation may be any one of the coefficients of determination ($R_x^2$), significance ($PVAL_x$), and mean absolute error ($MAE_x$)

Thereafter, the method predicts the subject's age based on the analysis result of the association analysis unit 140 and the data for each omics region in the age prediction unit 170 (S750).

Process S750 may be a process of predicting the subject's age by integrating (summing) the analysis result data for each of the plurality of types of analyzed omics areas.

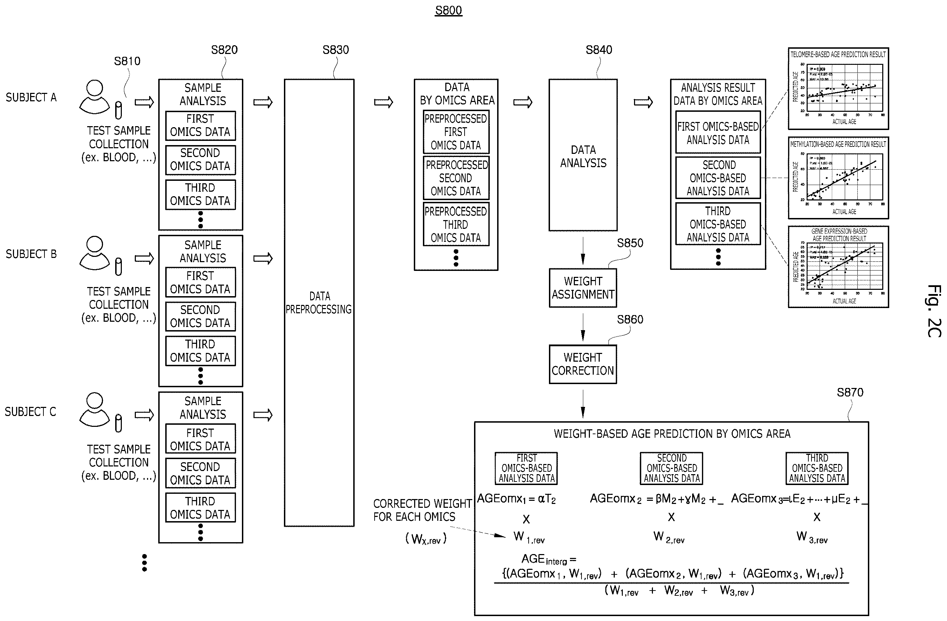

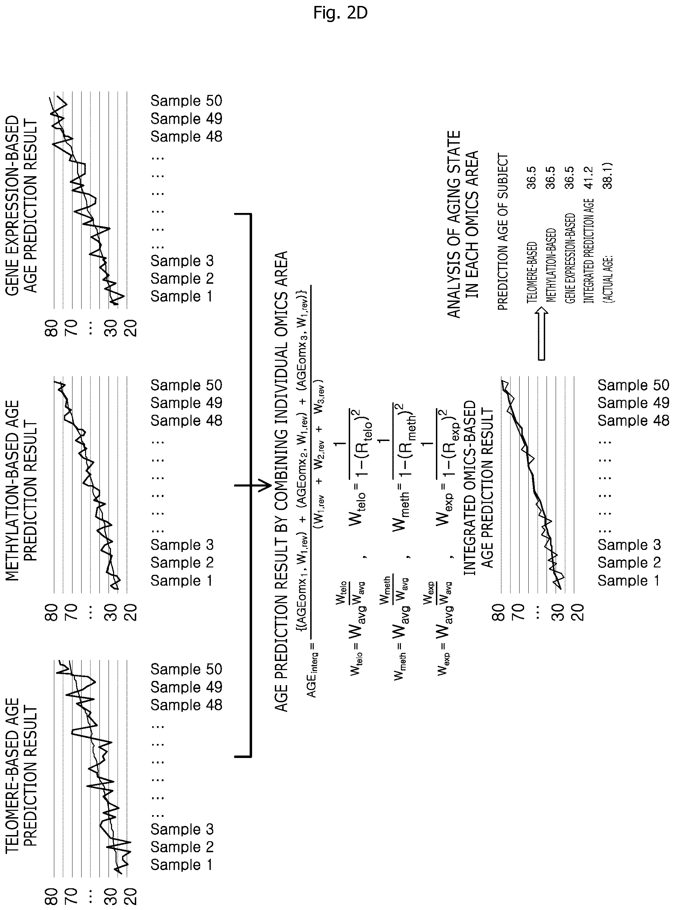

Hereinafter, a method for predicting biological age based on various omics data analysis according to the second embodiment of the present invention is described with reference to Figs. 2c and 2d.

The method S800 for predicting biological age based on various omics data analysis according to an embodiment of the present invention collects a plurality of genetic test samples, including DNA and RNA of a subject in the test sample collection unit 110 (S810), then analyzes a plurality of types of omics data (including at least one of telomere length, methylation, and gene expression) from each of the plurality of genetic test samples in the test sample analysis unit 120 (S820), and then preprocesses conversion of each marker value of the omics data analyzed through the test sample analysis unit 120 into a percentile value in the range of 0 to 1 in a preprocessing unit 130 (S830).

Thereafter, the method performs an association analysis based on the type of omics data for each omics area converted through the preprocessing unit 130 in the association analysis unit 140 (S840).

Process S840 is a process of analyzing the correlation between data for a plurality of omics areas using multiple linear regression analysis or artificial neural network-based regression analysis in which the analyzed correlation may be any one of the coefficients of determination ( $R_x^2$ ), significance ($PVAL_x$), and mean absolute error ($MAE_x$)

When process S840 is completed, the method assigns a weight to each type of omics data based on any one of the associations (coefficient of determination, significance, and mean absolute error) analyzed through the association analysis unit in the weight allocation unit 150 (S850).

Here, the weight allocation unit 150 calculates the weight ($W_x$) for the coefficient of determination ( $R_x^2$ ), significance ($PVAL_x$), and mean absolute error ($MAE_x$) of each omics area using the following equations.

$$W_x = 1/(1 - R_x^2) \dots\dots (\text{Weight equation for coefficient of determination})$$

$$W_x = \log(PVAL_x) * (-1) \dots\dots (\text{Weight equation for significance})$$

$$W_x = 1/\text{mae}_{x,\text{rev}} \dots\dots (\text{Weight equation for mean absolute error})$$

When process S850 is completed, the weight correction unit 160 may be configured to obtain a correction weight ($W_{x,rev}$) by exponentiating a weighted average value ($W_{avg}$) for each area of the weights given to each type of omics data using the following equations (S760).

$$W_{x,rev} = W_{avg}^{(W_x/W_{avg})}$$

Meanwhile, the weight correction unit 160 may obtain distribution correction ($mae_{x,rev}$) by the average age ($AGE_{avg}$) of the sample group to relatively reflect the mean absolute error compared to the actual age distribution before weight correction for the mean absolute error ($MAE_x$) of each omics area through the following equation.

$$\text{mae}_{x,rev} = MAE_x/AGE_{avg}$$

When process S860 is completed, the age prediction unit 170 predicts the subject's age based on the analysis result of the association analysis unit and the data for each omics area, and the subject's age is predicted through the following equation (S870).

$$AGE_{integ} = \frac{\Sigma)AGE_x * W_{x,rev})}{\Sigma W_{x,rev}}$$

(Biological age AGE<sub>integ</sub> prediction equation)

Therefore, an embodiment of the present invention is used to combine and reflect markers of several omics areas in the biological age prediction model, thereby offsetting errors existing in individual omics area and allowing more accurate biological age prediction and interpreting them by dividing the influence (or aging state) of each omics area with respect to the predicted biological age (the current aging state of the subject).

For example, through a combination of three omics data, including the genome (telomere length), exogenous (methylation), and transcript (gene expression) of samples such as human blood, 1) the age prediction accuracy can be improved by canceling the noise inherent in each omics data, and 2) the biological age (degree of aging) of the subject can be analyzed separately for each omics.

The above description of the present invention is for illustration, and those of ordinary skill in the art to which the present invention pertains can understand that it can be easily modified into other specific forms without changing the technical spirit or essential features of the present invention. Therefore, it should be understood that the embodiments described above are illustrative in all respects and not restrictive. For example, each component described as a single type may be implemented in a dispersed form, and likewise components described as distributed may be implemented in a combined form.

The scope of the present invention is indicated by the following claims rather than the above detailed description, and all changes or modifications derived from the meaning and scope of the claims and their equivalents should be construed as being included in the scope of the present invention.

[Description of reference numbers]

100: system
110: test sample collection unit
120: test sample analysis unit
130: preprocessing unit
140: association analysis unit
150: weight allocation unit
160: weight correction unit
170: age prediction unit

**Claims**

1. A system for predicting biological age based on various omics data analysis, the system comprising:

   a test sample collection unit for collecting a plurality of genetic test samples including at least one of DNA and RNA of a subject;
   a test sample analysis unit for analyzing a plurality of types of omics data from each of the plurality of genetic test samples;
   a preprocessing unit for preprocessing the omics data analyzed through the test sample analysis unit;
   an association analysis unit for performing an association analysis based on the omics type of data for each omics area converted through the preprocessing unit; and
   an age prediction unit for predicting the age of the subject based on the analyzed result of the association analysis unit and the data for each omics area.

2. The system of claim 1, wherein the plurality of types of omics data comprises at least one of telomere length, methylation, and gene expression.

3. The system of claim 1, wherein the preprocessing unit converts each marker value of the plurality of types of omics data into a percentile value in the range of 0 to 1.

4. The system of claim 1, wherein the association analysis unit uses any one of multiple linear regression analysis and artificial neural network-based regression analysis to analyze at least one of the coefficient of determination ($R_x^2$), significance ($PVAL_x$), and mean absolute error ($MAE_x$) of a plurality of omics regions.

5. The system of claim 1, wherein the age prediction unit predicts the age of the subject by integrating (summing) the analysis result data for each of a plurality of types of omics areas analyzed by the association analysis unit.

6. The system of claim 1, comprising:

   a weight allocation unit in which a weight is assigned to each type of omics data based on the coefficient of determination ($R_x^2$) analyzed through the association analysis unit; and
   a weight correction unit for correcting weights assigned to each type of omics data.

7. The system of claim 1, further comprising:

   a weight allocation unit in which a weight is assigned to each type of omics data based on the significance ($PVAL_x$) analyzed through the association analysis unit; and
   a weight correction unit for correcting weights assigned to each type of omics data.

**8.** The system of claim 1, further comprising:

a weight allocation unit in which a weight is assigned to each type of omics data based on the mean absolute error ($MAE_x$) analyzed through the association analysis unit; and
a weight correction unit for correcting weights assigned to each type of omics data.

**9.** The system of any one of claims 6 to 8, wherein the age prediction unit predicts the age of the subject using the following equation based on the weight corrected through the weight correction unit, the analysis result of the association analysis unit, and the data for each omics area,

[Equation]

$$AGE_{integ} = \frac{\Sigma)AGE_x * W_{x,rev})}{\Sigma W_{x,rev}}$$

**10.** A method for predicting biological age based on various omics data analysis, the method comprising steps of:

collecting a plurality of genetic test samples including at least one of DNA and RNA of a subject in a test sample collection unit;
analyzing a plurality of types of omics data from each of the plurality of genetic test samples in a test sample analysis unit;
preprocessing the omics data analyzed through the test sample analysis unit in a preprocessing unit;
performing an association analysis based on each omics type of data for each omics area converted through the preprocessing unit in an association analysis unit; and
predicting the age of a subject based on the analysis result of the association analysis unit and the data for each omics area in the age prediction unit.

**11.** The method of claim 10, wherein the plurality of types of omics data comprises at least one of telomere length, methylation, and gene expression.

**12.** The method of claim 10, wherein the step of preprocessing converts each marker value of the plurality of types of omics data into a percentile value in the range of 0 to 1.

**13.** The method of claim 10, wherein the step of association analysis uses any one of multiple linear regression analysis and artificial neural network-based regression analysis to analyze at least one of the coefficient of determination ($R_x^2$), significance ($PVAL_x$), and mean absolute error ($MAE_x$) of a plurality of omics regions.

**14.** The method of claim 10, wherein the step of predicting an age predicts the age of the subject by integrating (summing) the analysis result data for each of a plurality of types of omics areas analyzed by the association analysis unit.

**15.** The method of claim 10, comprising:

assigning a weight to each type of omics data based on the coefficient of determination ($R_x^2$) analyzed through the association analysis unit in a weight allocation unit; and
correcting weights assigned to each type of omics data in a weight correction unit.

**16.** The method of claim 10, further comprising:

assigning a weight to each type of omics data based on the significance ($PVAL_x$) analyzed through the association analysis unit in a weight allocation unit; and
correcting weights assigned to each type of omics data in a weight correction unit.

**17.** The method of claim 10, further comprising:

assigning a weight to each type of omics data based on the mean absolute error ($MAE_x$) analyzed through the

association analysis unit in a weight allocation unit to; and
correcting weights assigned to each type of omics data in a weight correction unit.

**18.** The method of any one of claims 15 to 17, wherein the age of the subject is predicted in an age prediction unit using the following equation based on the weight corrected through the weight correction unit, the analysis result of the association analysis unit, and the data for each omics area,

[Equation]

$$AGE_{integ} = \frac{\sum)AGE_{\chi} * W_{\chi,rev})}{\sum W_{\chi,rev}}$$

Fig. 1

100

```
┌─────────────────────┐
│    TEST SAMPLE      │
│  COLLECTION UNIT    │───110
└─────────────────────┘
          │
          ▼
┌─────────────────────┐
│    TEST SAMPLE      │
│   ANALYSIS UNIT     │───120
└─────────────────────┘
          │
          ▼
┌─────────────────────┐
│   PREPROCESSING     │
│       UNIT          │───130
└─────────────────────┘
          │
          ▼
┌─────────────────────┐
│    ASSOCIATION      │
│   ANALYSIS UNIT     │───140
└─────────────────────┘
          │
          ▼
┌─────────────────────┐
│      WEIGHT         │
│  ALLOCATION UNIT    │───150
└─────────────────────┘
          │
          ▼
┌─────────────────────┐
│      WEIGHT         │
│  CORRECTION UNIT    │───160
└─────────────────────┘
          │
          ▼
┌─────────────────────┐
│   AGE PREDICTION    │
│       UNIT          │───170
└─────────────────────┘
```

Fig. 2A

Fig. 2B

S800

S810　　　S820　　　　　S830　　　　　　　　　　　　　S840

SUBJECT A

TEST SAMPLE
COLLECTION
(ex. BLOOD, ...)

SAMPLE
ANALYSIS

FIRST
OMICS DATA

SECOND
OMICS DATA

THIRD
OMICS DATA

DATA
PREPROCESSING

DATA
BY OMICS AREA

PREPROCESSED
FIRST
OMICS DATA

PREPROCESSED
SECOND
OMICS DATA

PREPROCESSED
THIRD
OMICS DATA

DATA
ANALYSIS

ANALYSIS RESULT
DATA BY OMICS AREA

FIRST OMICS-BASED
ANALYSIS DATA

SECOND
OMICS-BASED
ANALYSIS DATA

THIRD
OMICS-BASED
ANALYSIS DATA

TELOMERE-BASED AGE PREDICTION RESULT

METHYLATION-BASED AGE PREDICTION RESULT

GENE EXPRESSION-BASED
AGE PREDICTION RESULT

SUBJECT B

TEST SAMPLE
COLLECTION
(ex. BLOOD, ...)

SAMPLE
ANALYSIS

FIRST
OMICS DATA

SECOND
OMICS DATA

THIRD
OMICS DATA

S850

WEIGHT
ASSIGNMENT

S860

WEIGHT
CORRECTION

SUBJECT C

TEST SAMPLE
COLLECTION
(ex. BLOOD, ...)

SAMPLE
ANALYSIS

FIRST
OMICS DATA

SECOND
OMICS DATA

THIRD
OMICS DATA

S870

WEIGHT-BASED AGE PREDICTION BY OMICS AREA

FIRST
OMICS-BASED
ANALYSIS DATA

SECOND
OMICS-BASED
ANALYSIS DATA

THIRD
OMICS-BASED
ANALYSIS DATA

CORRECTED WEIGHT
FOR EACH OMICS

$( W_{x,rev} )$

$AGEomx_1 = \alpha T_2$

$\times$

$W_{1,rev}$

$AGEomx_2 = \beta M_2 + \gamma M_2 + \_$

$\times$

$W_{2,rev}$

$AGEomx_3 = \iota E_2 + \cdots + \mu E_2 + \_$

$\times$

$W_{3,rev}$

$$AGE_{interg} = \frac{\{(AGEomx_1, W_{1,rev}) + (AGEomx_2, W_{1,rev}) + (AGEomx_3, W_{1,rev})\}}{(W_{1,rev} + W_{2,rev} + W_{3,rev})}$$

Fig. 2C

TELOMERE-BASED AGE
PREDICTION RESULT

METHYLATION-BASED AGE
PREDICTION RESULT

GENE EXPRESSION-BASED
AGE PREDICTION RESULT

AGE PREDICTION RESULT BY COMBINING INDIVIDUAL OMICS AREA

$$AGE_{interg} = \frac{\{(AGEomx_1, W_{1,rev}) + (AGEomx_2, W_{1,rev}) + (AGEomx_3, W_{1,rev})\}}{(W_{1,rev} + W_{2,rev} + W_{3,rev})}$$

$$W_{telo} = W_{avg}\frac{W_{telo}}{W_{avg}} \quad , \quad W_{telo} = \frac{1}{1-(R_{telo})^2}$$

$$W_{meth} = W_{avg}\frac{W_{meth}}{W_{avg}} \quad , \quad W_{meth} = \frac{1}{1-(R_{meth})^2}$$

$$W_{exp} = W_{avg}\frac{W_{exp}}{W_{avg}} \quad , \quad W_{exp} = \frac{1}{1-(R_{exp})^2}$$

INTEGRATED OMICS-BASED
AGE PREDICTION RESULT

ANALYSIS OF AGING STATE
IN EACH OMICS AREA

PREDICTION AGE OF SUBJECT

| | |
|---|---|
| TELOMERE-BASED | 36.5 |
| METHYLATION-BASED | 36.5 |
| GENE EXPRESSION-BASED | 36.5 |
| INTEGRATED PREDICTION AGE | 41.2 |
| (ACTUAL AGE: | 38.1) |

Fig. 2D

21

Fig. 3

TELOMERE-BASED AGE PREDICTION RESULT

$R^2 = 0.317$
P.val = 1.7E−05
MAE = 10.593

PREDICTION AGE

ACTUAL AGE

Fig. 4

METHYLATION-BASED AGE PREDICTION RESULT

$R^2 = 0.930$
P.val = 6.4E−30
MAE = 3.003

PREDICTION AGE

ACTUAL AGE

Fig. 5

GENE EXPRESSION-BASED
AGE PREDICTION RESULT

$R^2 = 0.834$
P.val = 9.9E−21
MAE = 5.094

Fig. 6

INTEGRATED OMICS-BASED
AGE PREDICTION RESULT

$R^2 = 0.979$
P.val = 6.5E−43
MAE = 1.773

Fig. 7

OMICS AGE SUMMATION RESULT
(COEFFICIENT OF DETERMINATION WEIGHTED)

R² = 0.936
P.val = 7.2E-31
MAE = 2.934

PREDICTION AGE

ACTUAL AGE

Fig. 8

METHYLATION-BASED AGE PREDICTION RESULT

R² = 0.860
P.val = 1.5E-22
MAE = 4.637

PREDICTION AGE

ACTUAL AGE

Fig. 9

GENE EXPRESSION-BASED
AGE PREDICTION RESULT

$R^2 = 0.717$
P.val = 4.8E-15
MAE = 6.263

PREDICTION AGE

ACTUAL AGE

Fig. 10

INTEGRATED OMICS-BASED
AGE PREDICTION RESULT

$R^2 = 0.887$
P.val = 8.2E-25
MAE = 4.052

PREDICTION AGE

ACTUAL AGE

Fig. 11

**OMICS AGE SUMMATION RESULT
(COEFFICIENT OF DETERMINATION WEIGHTED)**

Fig. 12

**OMICS AGE SUMMATION RESULT
(COEFFICIENT OF DETERMINATION WEIGHTED)**

Fig. 13

METHYLATION-BASED AGE PREDICTION RESULT

$R^2 = 0.969$
P.val = 1.1E-38
MAE = 1.71

PREDICTION AGE / ACTUAL AGE

Fig. 14

GENE EXPRESSION-BASED
AGE PREDICTION RESULT

$R^2 = 0.959$
P.val = 1.1E-35
MAE = 2.29

PREDICTION AGE / ACTUAL AGE

Fig. 15

INTEGRATED OMICS-BASED
AGE PREDICTION RESULT

R² = 0.980
P.val = 2.3E-43
MAE = 1.39

PREDICTION AGE

ACTUAL AGE

Fig. 16

OMICS AGE SUMMATION RESULT
(COEFFICIENT OF DETERMINATION WEIGHTED)

R² = 0.971
P.val = 2.96E-39
MAE = 1.67

PREDICTION AGE

ACTUAL AGE

Fig. 17

OMICS AGE SUMMATION RESULT
(SIGNIFICANCE WEIGHTED)

$R^2$ = 0.938
P.val = 3.3E−31
MAE = 2.991

PREDICTION AGE / ACTUAL AGE

Fig. 18

OMICS AGE SUMMATION RESULT
(MEAN ERROR WEIGHTED)

$R^2$ = 0.938
P.val = 2.9E−31
MAE = 2.987

PREDICTION AGE / ACTUAL AGE

## INTERNATIONAL SEARCH REPORT

| International application No. |
|---|
| **PCT/KR2021/004293** |

| **A.** | **CLASSIFICATION OF SUBJECT MATTER** |
|---|---|

**G16B 40/20**(2019.01)i; **G16B 20/40**(2019.01)i

According to International Patent Classification (IPC) or to both national classification and IPC

| **B.** | **FIELDS SEARCHED** |
|---|---|

Minimum documentation searched (classification system followed by classification symbols)

G16B 40/20(2019.01); C12Q 1/68(2006.01); G01N 33/52(2006.01); G06F 19/00(2011.01); G16B 25/10(2019.01); G16B 40/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 오믹스 데이터 분석(omics data analysis), 생체나이(biological age), 예측(prediction), 연관성(association)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | SOLOVEV, I. et al. Multi-omics approaches to human biological age estimation. Mechanisms of Ageing and Development. electronic publication 28 November 2019, vol. 185, document 111192, pp. 1-9. See abstract; table 1; and Conclusion. | 1-18 |
| A | ZIERER, J. et al. Integration of 'omics' data in aging research: from biomarkers to systems biology. Aging Cell. 2015, vol. 14, pp. 933-944. See entire document. | 1-18 |
| A | KR 10-2015-0061914 A (KOREA UNIVERSITY RESEARCH AND BUSINESS FOUNDATION) 05 June 2015 (2015-06-05) See entire document. | 1-18 |
| A | KR 10-2012-0009230 A (GYEONGNAM NATIONAL UNIVERSITY OF SCIENCE AND TECHNOLOGY INDUSTRY-ACADEMIC COOPERATION FOUNDATION) 01 February 2012 (2012-02-01) See entire document. | 1-18 |

| ☑ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 July 2021** | **21 July 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/004293** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2019-0272890 A1 (INSILICO MEDICINE, INC.) 05 September 2019 (2019-09-05)<br>See entire document. | 1-18 |
| A | GOEL, N. et al. Role of DNA methylation in human age prediction. Mechanisms of Ageing and Development. 2017, vol. 166, pp. 33-41.<br>See entire document. | 1-18 |
| A | PYRKOV, T. V. et al. Extracting biological age from biomedical data via deep learning: too much of a good thing?. Scientific Reports. 2018, vol. 8, document 5210, pp. 1-11.<br>See entire document. | 1-18 |

Form PCT/ISA/210 (second sheet) (July 2019)

**INTERNATIONAL SEARCH REPORT**
Information on patent family members

International application No.

**PCT/KR2021/004293**

| Patent document cited in search report | | | Publication date (day/month/year) | Patent family member(s) | | | Publication date (day/month/year) |
|---|---|---|---|---|---|---|---|
| KR | 10-2015-0061914 | A | 05 June 2015 | KR | 10-1538231 | B1 | 20 July 2015 |
| KR | 10-2012-0009230 | A | 01 February 2012 | None | | | |
| US | 2019-0272890 | A1 | 05 September 2019 | US | 10325673 | B2 | 18 June 2019 |
| | | | | US | 10665326 | B2 | 26 May 2020 |
| | | | | US | 2019-0030078 | A1 | 31 January 2019 |
| | | | | US | 2019-0034581 | A1 | 31 January 2019 |
| | | | | US | 2020-0075127 | A1 | 05 March 2020 |
| | | | | US | 2020-0286625 | A1 | 10 September 2020 |
| | | | | WO | 2020-084536 | A1 | 30 April 2020 |

Form PCT/ISA/210 (patent family annex) (July 2019)

**EP 4 138 083 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030148350 A1